# EUROPÄISCHE PATENTANMELDUNG

(11) **EP 4 606 714 A1**
(43) Veröffentlichungstag der Anmeldung: **27.08.2025**
(21) Anmeldenummer: 24159466.2
(22) Anmeldetag: 23.02.2024
(51) Int. Cl.: B65B 11/50, B65B 47/06, B65B 51/30

(54) **VORRICHTUNG UND VERFAHREN ZUM EINSIEGELN EINES KATHETER-TEILS IN EINE FOLIENMANSCHETTE**

(71) Anmelder: Harro Höfliger Verpackungsmaschinen GmbH, 71573 Allmersbach im Tal (DE)
(72) Erfinder: Signore, Andreas, 71573 Allmersbach im Tal (DE); Schwarz, Kevin, 71573 Allmersbach im Tal (DE); Müller, Maik, 71573 Allmersbach im Tal (DE)
(74) Vertreter: Zurhorst, Stefan

(57) **Zusammenfassung**

Die Erfindung betrifft eine Vorrichtung und ein Verfahren zum Einsiegeln eines Katheter-Teils (20) in eine Folienmanschette (21). Formplatten (6, 9) sind mit Formmulden (7, 10) versehen, welche eine eindimensional gekrümmte Querschnittsform aufweisen. In Formstationen (1, 2) werden Basis- und Deckfolienabschnitte (15, 16) in die Formmulden (7, 10) dehnungsfrei eingelegt. In einer Einführstation (3) wird mindestens ein Katheter-Teil (20) in eine korrespondierende Formmulde (7) eingeführt. In einer Siegelstation (4) wird das Katheter-Teil (20) in eine Folienmanschette (21) eingesiegelt, indem der Basisfolienabschnitt (15) und der Deckfolienabschnitt (16) an gemeinsamen Siegelabschnitten (17) aufeinander gesiegelt und auf Siegelflächen (22) des Katheter-Teils (20) unter Bildung der Folienmanschette (21) aufgesiegelt werden.

## Beschreibung

Die Erfindung betrifft eine Vorrichtung und ein Verfahren zum Einsiegeln von zumindest einem Katheter-Teil in eine Folienmanschette.

Bei Kathetern insbesondere für medizinische Anwendungen besteht die Anforderung, diese vor dem Gebrauch steril zu lagern. Hierzu eignen sich insbesondere Folienschläuche bzw. Folienmanschetten, in welche ein solcher Katheter ganz oder in Teilen eingesiegelt werden kann. Problematisch ist dabei die Bildung einer Folienmanschette mit einer bestimmten Maßgenauigkeit, wobei auch hohe Anforderungen an die Dichtigkeit insbesondere an den Siegelstellen zu erfüllen sind.

Der Erfindung liegt die Aufgabe zugrunde, eine Vorrichtung anzugeben, welche eine zuverlässige, wiederholgenaue Bildung von Folienmanschetten mit hoher Qualität insbesondere hinsichtlich Manschettenvolumen und Dichtigkeit und eine Einsiegelung des Katheter-Teils mit entsprechender Qualität ermöglicht.

Diese Aufgabe wird durch eine Vorrichtung mit den Merkmalen des Anspruchs 1 gelöst.

Der Erfindung liegt des Weiteren die Aufgabe zugrunde, ein entsprechendes Verfahren anzugeben, mit dem ein hochqualitatives Einsiegeln möglich ist.

Diese Aufgabe wird durch ein Verfahren mit den Merkmalen des Anspruchs 10 gelöst.

Der gewählte und hier im Zusammenhang mit der Erfindung verwendete Begriff "Katheter-Teil" umfasst einen Katheter in seiner Gesamtheit sowie auch Teile bzw. Komponenten davon wie Anschlussteile oder dergleichen.

Nach der Erfindung ist eine Vorrichtung zum Einsiegeln von zumindest einem Katheter-Teil in eine Folienmanschette vorgesehen, welche eine erste Formstation, eine zweite Formstation, eine Einführstation zum Einführen des Katheter-Teils und eine Siegelstation zum Aufsiegeln eines Basisfolienabschnitts und eines Deckfolienabschnitts aufeinander und auf Siegelflächen des Katheter-Teils unter Bildung der Folienmanschette umfasst. Die Vorrichtung umfasst darüber hinaus eine erste Formplatte mit mindestens einer ersten Formmulde für den Basisfolienabschnitt und eine zweite Formplatte mit mindestens einer zweiten Formmulde für den Deckfolienabschnitt, wobei die erste Formmulde und die zweite Formmulde im Bereich des Basisfolienabschnitts bzw. des Deckfolienabschnitts eine eindimensional gekrümmte Querschnittsform aufweisen. Außerdem umfasst die Vorrichtung erste Einlegemittel zum dehnungsfreien Einlegen des Basisfolienabschnitts in die mindestens eine erste Formmulde, sowie zweite Einlegemittel zum dehnungsfreien Einlegen des Deckfolienabschnitts in die mindestens eine zweite Formmulde.

Im entsprechenden erfindungsgemäßen Verfahren werden folgende Verfahrensschritte ausgeführt:
- In der ersten Formstation wird ein Basisfolienabschnitt in mindestens eine erste Formmulde einer ersten Formplatte dehnungsfrei eingelegt,
- In einer Einführstation wird mindestens ein Katheter-Teil bei eingelegtem Basisfolienabschnitt in eine korrespondierende erste Formmulde eingeführt,
- In der zweiten Formstation wird ein Deckfolienabschnitt in mindestens eine zweite Formmulde einer zweiten Formplatte dehnungsfrei eingelegt,
- In der Siegelstation wird das Katheter-Teil in eine Folienmanschette eingesiegelt, indem die erste Formplatte und die zweite Formplatte bei eingelegtem Basisfolienabschnitt, bei eingelegtem Deckfolienabschnitt und bei eingeführtem Katheter-Teil zusammengeführt werden, wobei der Basisfolienabschnitt und der Deckfolienabschnitt an gemeinsamen Siegelabschnitten aufeinander gesiegelt und auf Siegelflächen des Katheter-Teils unter Bildung der Folienmanschette aufgesiegelt werden.

Dabei beruht die Erfindung auf dem Prinzip, Folienabschnitte zu einer Folienmanschette zu formen, ohne dabei - im Unterschied beispielsweise zum Tiefziehverfahren - das flächige Folienmaterial in der Materialebene einer Dehn- und/oder einer Schubverformung zu unterziehen. Insbesondere findet bei der Formung in den Formmulden keine plastische Verformung des Folienmaterials statt. Vielmehr führt die eindimensional gekrümmte Querschnittsform der Formmulden zu einer ebensolchen Krümmung des jeweiligen Basisfolienabschnitts bzw. des Deckfolienabschnitts. Eine eindimensionale Krümmung ist eine Krümmung ohne sphärische Krümmungsanteile, also eine solche Krümmung, wie sie beispielsweise ein Blatt Papier einnehmen kann, ohne zu knittern. Die Verformung des Folienmaterials reduziert sich auf eine reine Biegeverformung. Da die Dicke der zum Einsatz kommenden Folien gering ist im Vergleich zu ihren übrigen Abmessungen, sind die entsprechenden Biegeverformungen und die daraus resultierenden Dehnungsanteile im technischen Sinne allenfalls geringfügig und im Wesentlichen vernachlässigbar. Insgesamt findet also im technischen Sinne ein dehnungsfreies Einlegen in die Form und eine dehnungsfreie Formgebung der Folienabschnitte statt. Dies führt zu einer hohen Maßhaltigkeit und Wiederholgenauigkeit. Die faltenfrei und praktisch spannungsfrei in Form gebrachten Folienabschnitte können exakt aufeinander sowie auf entsprechende Siegelbereiche des Katheter-Teils aufgesiegelt werden, sodass die geforderte Dichtigkeit mit hoher Zuverlässigkeit erreicht werden kann.

Es kann zweckmäßig sein, in den einzelnen Formplatten nur jeweils eine einzige Formmulde auszubilden. In einer bevorzugten Ausführungsform sind in der ersten Formplatte mehrere erste Formmulden und in der zweiten Formplatte eine korrespondierende Anzahl von zweiten Formmulden ausgebildet. Dies erlaubt die Formgebung, das Einsetzen von Teilen, das Siegeln und ggf. auch das Vereinzeln mehrerer Einheiten in nur jeweils einer zugeordneten Station.

Das Einlegen der Folienabschnitte in die Formmulden kann beispielsweise durch eine geeignete Vakuumführung erfolgen. Vorteilhaft sind die ersten Einlegemittel und/oder die zweiten Einlegemittel als mit den ersten bzw. zweiten Formmulden korrespondierende Druckstempel ausgeführt. Hierdurch kann mit einfachen Mitteln ein exaktes Einlegen der Folienabschnitte in die Formmulden erreicht werden. Durch eine entsprechende elastische Lagerung und/oder Bewegungssteuerung kann ein sequentielles Einlegen in mehrere Formmulden einer Formplatte vorgenommen werden, was ein dehnungsfreies Nachführen einer ausreichenden Folienmenge ermöglicht.

In zweckmäßiger Weiterbildung sind die ersten und/oder zweiten Formplatten mit Haltemitteln zum Halten des eingelegten Basisfolienabschnitts in der ersten Formmulde bzw. des eingelegten Deckfolienabschnitts in der zweiten Formmulde versehen. Dabei kann es sich zumindest für einen Teil des Prozesses um die vorgenannten Einlegemittel bzw. Druckstempel handeln. Bevorzugt sind die Haltemittel Form einer Unterdruckzufuhr ausgeführt. Die Unterdruckzufuhr stellt bei anliegendem Unterdruck sicher, dass die Folienabschnitte sauber an der Oberfläche der Formmulden anliegen. Gleichzeitig besteht hierfür innerhalb der Formmulden kein Platzbedarf, sodass das entsprechende Katheter-Teil ungestört eingelegt und eingesiegelt werden kann.

Im Rahmen der Erfindung kommen verschiedene Querschnittsformen der Formmulden in Betracht. Bevorzugt sind die ersten und/oder zweiten Formmulden in Form eines Halbzylinders ausgeführt. Bei entsprechend zylindrischer Ausformung des Katheter-Teils kommt es beim Einführen und Einsiegeln auf dessen Drehwinkellage nicht an, so dass der Prozess vereinfacht ist.

Für die kontinuierliche oder getaktete Verarbeitung der Folien kommen kontinuierliche Folienbahnen, vereinzelte Folienblätter oder Kombinationen davon in Betracht. In einer bevorzugten Ausführungsform sind Schneidmittel zur Vereinzelung des Basisfolienabschnitts bzw. des Deckfolienabschnitts aus einer kontinuierlichen Folienbahn vorgesehen und insbesondere in zumindest einer der beiden Formstationen angeordnet. Die Erzeugung und Verwendung vereinzelter Folienblätter erzeugt zusätzliche Bewegungsmöglichkeiten der Komponenten einzelner Bearbeitungsstationen. Insbesondere kann eine entsprechend bestückte Formplatte leicht zur jeweils anderen Formplatte geführt werden, sodass beide in der Siegelstation zur Ausführung des Siegelvorganges aufeinandergepresst werden können.

Zweckmäßig ist in zumindest einer der beiden Formplatten eine Siegelheizung integriert. Die entsprechende Formplatte übt damit in Doppelfunktion nicht nur die Funktion der Formgebung, sondern auch die Funktion des Siegelns aus. Der Aufwand von beispielsweise separaten Siegelbacken entfällt dadurch.

Es kann im Rahmen der Erfindung vorteilhaft sein, eine durchgehende, zusammenhängende Bahn von eingesiegelten Katheter-Teilen herzustellen. In einer bevorzugten Ausführungsform umfasst die Vorrichtung eine Trennstation zum Vereinzeln von mehreren eingesiegelten Katheter-Teilen. Dadurch kann einerseits beim Formgeben, Einsetzen und Siegeln die Prozesssicherheit bei der Verarbeitung eines durchgehenden Produktstreifens genutzt werden, währen andererseits im Anschluss an die Trennstation einsatzfertige Einzelprodukte zur Verfügung stehen.

Ein Ausführungsbeispiel der erfindungsgemäßen Vorrichtung und des erfindungsgemäßen Verfahrens ist nachfolgend anhand der Zeichnung näher beschrieben. Es zeigen:
- Fig. 1: in einer perspektivischen Teilansicht eine erfindungsgemäß ausgeführte Vorrichtung mit zwei Formstationen, mit einer Einführstation, mit einer Siegelstation und mit einer Trennstation,
- Fig. 2: in einer schematischen Darstellung ein mit der Vorrichtung nach Fig. 1 in eine Folienmanschette eingesiegeltes Katheter-Teil,
- Fig. 3: in einer perspektivischen Schemadarstellung eine Formplatte für den Basis-folienabschnitt und eine Formplatte für den Deckfolienabschnitt,
- Fig. 4: in Einzeldarstellung die erste Formstation nach Fig. 1 mit einer Formplatte nach Fig. 3 und mit angesetzten Druckstempeln beim Einlegen des Basis-folienabschnitts,
- Fig. 5: in Einzeldarstellung die Einführstation nach Fig. 1 beim Einführen mehrerer Katheter-Teile,
- Fig. 6: in Detaildarstellung die zweite Formstation und die Siegelstation nach Fig. 1 beim Formen und Aufsiegeln des Deckfolienabschnitts, und
- Fig. 7: in perspektivischer Ansicht einen Teil der Trennstation nach Fig. 1 mit einer Reihe von Folienmessern beim Vereinzeln der eingesiegelten Katheter-Teile.

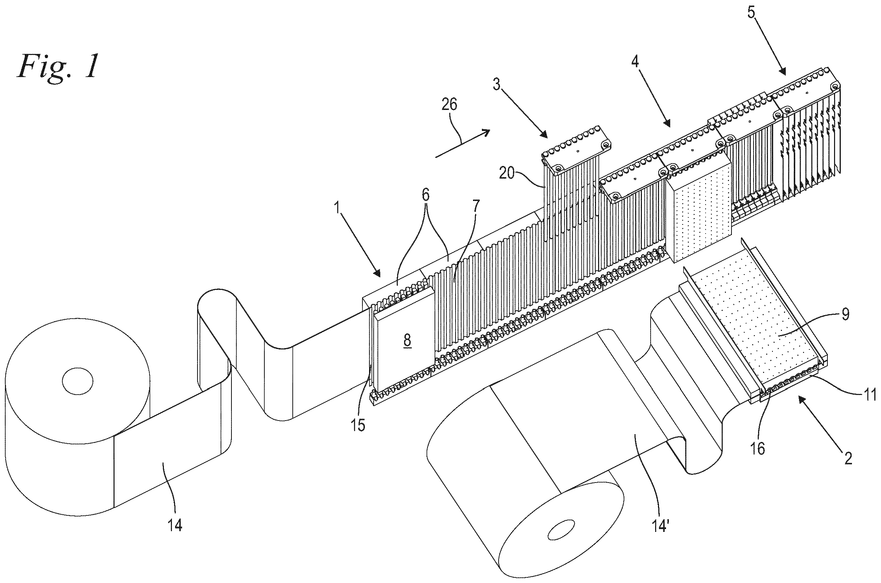

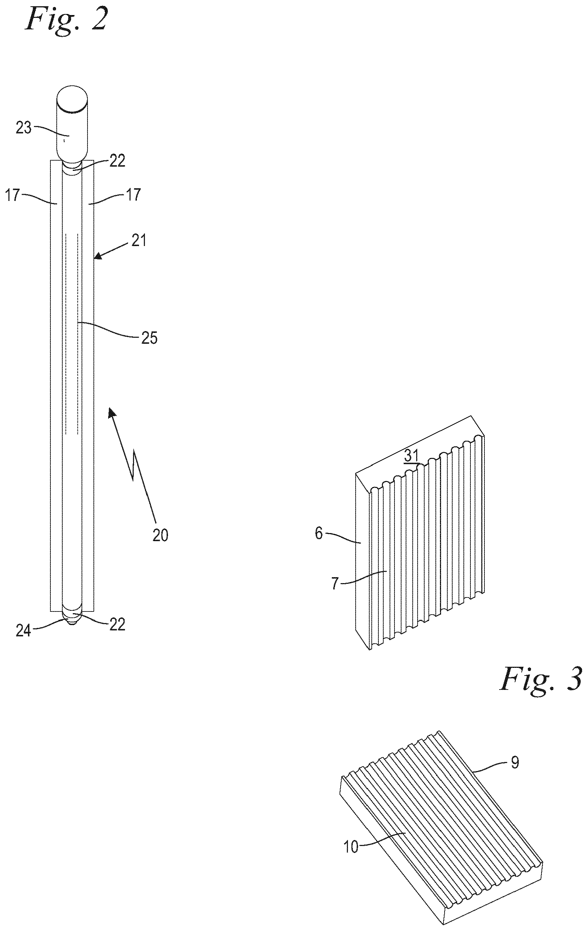

Fig. 1 zeigt in einer perspektivischen Teilansicht eine erfindungsgemäß ausgeführte Vorrichtung zum Einsiegeln eines in Fig. 2 dargestellten Katheter-Teils 20 in eine Folienmanschette 21. Unter Bezug auf Fig. 2 umfasst der hier gewählte Begriff "Katheter-Teil" 20 im gezeigten Ausführungsbeispiel eine vollständige Katheter-Einheit mit einer nur angedeuteten Katheter-Röhre 25, mit einem Anschlussstück 23 und mit einem Kopfstück 24. Im Rahmen der Erfindung kann das Katheter-Teil 20 aber auch nur durch ein oder mehrere Teile der genannten vollständigen Katheter-Einheit gebildet sein.

Das Katheter-Teil 20 ist in erfindungsgemäßer Weise teilweise in eine Folienmanschette 21 eingesiegelt. Dazu ist die Folienmanschette 21 aus einem Basisfolienabschnitt 15 und aus einem Deckfolienabschnitt 16 (Fig. 1) gebildet. Der Deckfolienabschnitt 16 ist entlang von zwei beidseitig der Katheter-Röhre 25 und parallel zueinander verlaufenden Siegelabschnitten 17 auf den Basisfolienabschnitt 15 aufgesiegelt, wodurch die schlauchförmige Folienmanschette 21 gebildet ist. Diese ist ihrerseits an ihren beiden Enden auf geschlossen umlaufende Siegelflächen 22 des Katheter-Teils 20 aufgesiegelt, sodass der von der Folienmanschette 21 umschlossene Bereich des Katheter-Teils 20 dicht gegen die Umwelt abgeschirmt ist und steril gehalten werden kann. Die Erfindung umfasst ebenso das Einsiegeln von Einzelteilen oder Baugruppen der Katheter-Einheit, also beispielsweise das Einsiegeln des Anschlussstücks 23, des Kopfstücks 24 und/oder weiterer Teile, welche hier im Kontext dann als Katheter-Teil 20 zu verstehen sind.

Unter erneutem Bezug auf Fig. 1 ist erkennbar, dass die erfindungsgemäße Vorrichtung als sogenannter Oval-Läufer ausgeführt ist. Hierbei wird eine Reihe von ersten Formplatten 6 auf einer geschlossen umlaufenden Bahn linear an verschiedenen Stationen vorbeigeführt und anschließend parallel dazu zurückbewegt. Die Bewegung kann intermittierend mit Stopps an den einzelnen Stationen oder aber auch kontinuierlich sein. Der Einfachheit halber ist hier nur der Abschnitt der Vorrichtung gezeigt, in welchem die ersten Formplatten 6 Basisfolienabschnitte 15 von einer kontinuierlichen Folienbahn 14 aufnehmen und gemeinsam mit diesen in Richtung eines Pfeiles 26 entlang verschiedener Stationen, hier der Reihenfolge nach entlang einer ersten Formstation 1, einer Einführstation 3, einer Siegelstation 4 und einer Trennstation 5 verfahren werden.

In der ersten Formstation 1 erfolgt eine Formgebung der Basisfolienabschnitte 15. In der nachfolgenden Einführstation 3 werden Katheter-Teile 20 in die vorbereiteten ersten Formplatten 6 eingeführt. Parallel dazu werden Deckfolienabschnitte 16 aus einer weiteren kontinuierlichen Folienbahn 14' gebildet, einer zweiten Formstation 2 zugeführt und dort in einer zweiten Formplatte 9 geformt. Die zweite Formplatte 9 macht nicht die vorstehend beschriebene Oval-Läufer-Bewegung der ersten Formplatten 6 mit, sondern wird zyklisch zwischen der zweiten Formstation 2 und der Siegelstation hin und her bewegt: Nach der Formung eines Deckfolienabschnitts 16 wird die zweite Formplatte 9 mit dem darin gehaltenen Deckfolienabschnitt 16 zur Siegelstation 4 bewegt und dort mit einer korrespondierenden ersten Formplatte 6 zusammengeführt. Dabei werden der jeweilige Basisfolienabschnitt 16 und der Deckfolienabschnitt 17 aufeinander gesiegelt und mit dem Katheter-Teil 20 versiegelt, sodass eine zunächst zusammenhängende Bahn von eingesiegelten Katheter-Teilen 20 entsteht. Währen die zweite, nun entleerte Formplatte 9 zurück zur zweiten Formstation 2 bewegt wird, wird die erste Formplatte mit einer darin gehaltenen zusammenhängenden Reihe von eingesiegelten Katheter-Teilen 20 weiter zur Trennstation 5 bewegt, wo schließlich eine Vereinzelung der Katheter-Teile 20 in der in Fig. 2 gezeigten Form erfolgt.

Fig. 3 zeigt in einer perspektivischen Schemadarstellung Details einer ersten Formplatte 6 für den Basisfolienabschnitt 15 und einer zweiten Formplatte 9 für den Deckfolienabschnitt 16. In der ersten Formplatte 6 ist mindestens eine erste Formmulde 7 ausgebildet. Vorliegend handelt es sich um mehrere, hier zehn parallel zueinander liegende erste Formmulden 7 in der ersten Formplatte. Das charakteristische Merkmal dieser ersten Formmulden 7 besteht darin, dass sie eine eindimensional gekrümmte Querschnittsform aufweisen. Diese kann elliptisch, oval, kegelstumpfförmig, oder sogar eckig sein. Im gezeigten bevorzugten Ausführungsbeispiel ist der Querschnitt halbkreisförmig. Mit anderen Worten ist die erste Formmulde 7 in Form eines Halbzylinders ausgeführt. Eine korrespondierende Formgebung des darin eingelegten Basisfolienabschnitts 15 erfolgt damit dehnungsfrei ohne elastische oder gar plastische Verformung des Folienmaterials in der Folienebene, also ohne Dehnung und/oder Schubverformung in der Folienebene. Analog das Gleiche gilt für eine korrespondierende Anzahl von zweiten Formmulden 10 in der zweiten Formplatte 16 und den darin geformten Deckfolienabschnitt 16.

In Fig. 3 ist noch angedeutet, dass in der ersten Formplatte 6 eine Siegelheizung 31 angeordnet ist, welche in der weiter unten näher beschriebenen Siegelstation 4 zum Einsatz kommt. Auch die zweite Formplatte 9 kann mit einer solchen Siegelheizung ausgestattet sein. Natürlich sind auch andere Formen der Siegelung im Rahmen der Erfindung denkbar.

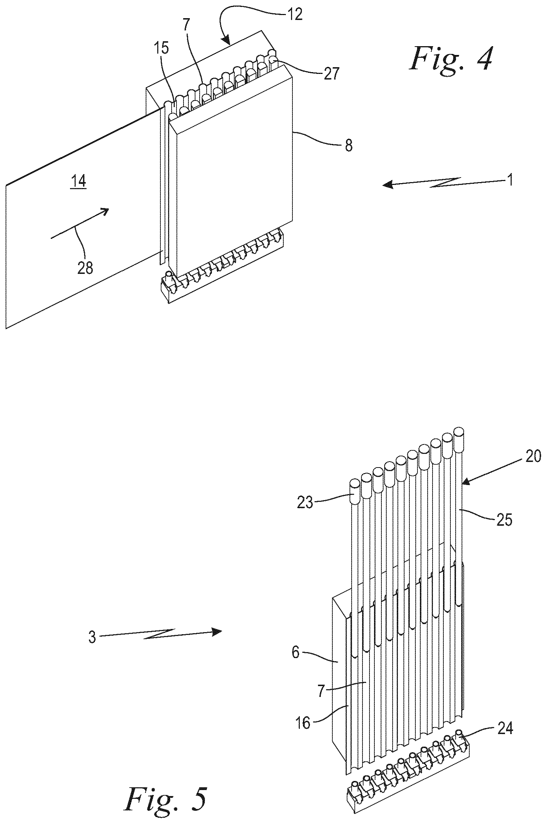

Fig. 4 zeigt in einer schematischen Einzeldarstellung die erste Formstation 1 nach Fig. 1 mit einer ersten Formplatte 6 nach Fig. 3. Dort wird zunächst ein Teil der kontinuierlichen Folienbahn 14 als Basisfolienabschnitt 15 auf die erste Formplatte 6 mit ihren ersten Formmulden 7 aufgelegt. Die einzelnen Basisfolienabschnitte 15 der einzelnen Formplatten 6 behalten dabei bis zur Trennstation 5 ihre Eigenschaft als zusammenhängende Folienbahn 14. Nach dem Auflegen des Basisfolienabschnitts 15 erfolgt dessen Formgebung durch dehnungsfreies Einlegen in die ersten Formmulden 7 mittels erster Einlegemittel 8.

Die ersten Einlegemittel 8 können eine gesteuerte Unterdruckzufuhr oder dergleichen sein und sind im gezeigten Ausführungsbeispiel als eine Reihe von Druckstempeln 27 ausgebildet, welche in Form, Lage und Anzahl mit den ersten Formmulden 7 korrespondieren. Es kann in Betracht kommen, den Basisfolienabschnitt 15 in sämtliche Formmulden 7 der Formplatte 6 gleichzeitig hineinzudrücken. Vorliegend geschieht dies sequentiell. In Fig 4 ist nämlich erkennbar, dass der im Bild rechte also von der Vorratsrolle der Folienbahn 14 am weitest entfernte Druckstempel 27 am weitesten von einer zugehörigen Grundplatte hervorsteht und in die entsprechende Formmulde hineinragt. Ausgehen hiervon springen die weiteren Druckstempel 27 treppenförmig zurück. Sämtliche Druckstempel 27 sind zudem elastisch nachgiebig gelagert. Es wird nun also die genannte Grundplatte auf die Formplatte 6 zubewegt. Dadurch tauchen die Druckstempel sequentiell nacheinander in die korrespondierenden Formmulden 7 ein. Beim Eintauchen drückt der jeweilige Druckstempel 27 den korrespondierenden Bereich des Basisfolienabschnitts 15 in die zugehörige Formmulde 7.

Damit der Basisfolienabschnitt dehnungsfrei der gekrümmten Querschnittskontur der Formmulden 7 folgen kann, ist eine größere Folienlänge erforderlich als die Länge des flach und unverformt auf der ersten Formplatte 6 aufliegenden Basisfolienabschnitts 15. Die hierfür erforderliche zusätzlich Folienmenge wird dehnungsfrei von der Folienbahn 14 entsprechend einem Pfeil 28 nachgeführt bzw. selbsttätig abgezogen, da die in Richtung der zugeführten Folienbahn 14 benachbarten Druckstempel 27 weniger tief eingeführt sind und deshalb eine Foliennachführung nicht behindern. Ausgehend von dem am weitesten hervorstehenden und deshalb zuerst eintauchenden Druckstempel kann auf diese Weise der Basisfolienabschnitt 15 sequentiell in sämtliche Formmulden 7 der ersten Formplatte eingedrückt werden, ohne dass eine technisch relevante Dehnung des Folienmaterials eintritt.

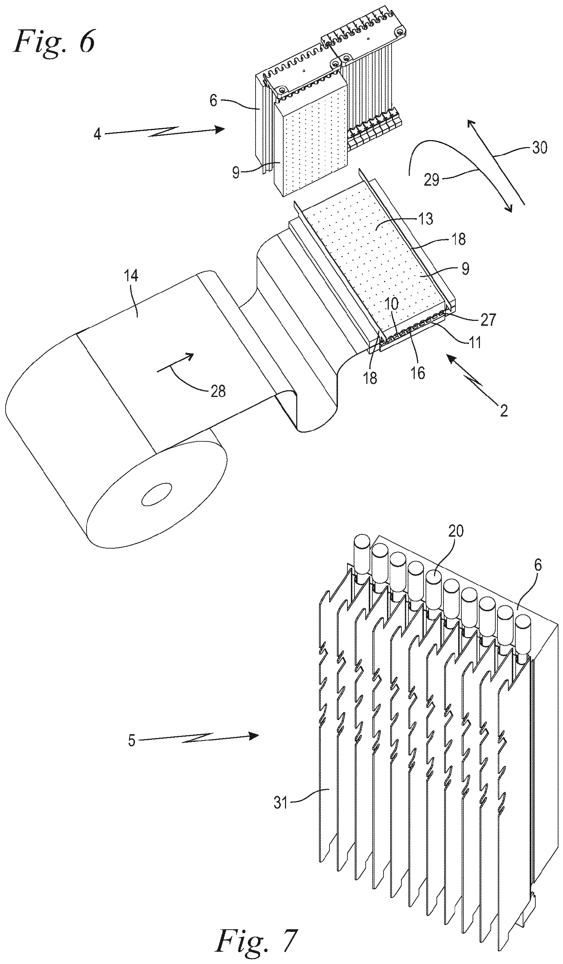

Darüber hinaus verfügt die erste Formplatte 6 noch über Haltemittel zum Halten des eingelegten Basisfolienabschnitts 15 in der ersten Formmulde 7, welche hier nicht näher dargestellt sind, aber welche analog zu Haltemitteln für den eingelegten Deckfolienabschnitt 16 in der zweiten Formmulde 10 gemäß Fig. 6 in Form einer Unterdruckzufuhr 12, 13 ausgeführt sind. Der in vorstehend beschriebener Weise geformte Basisfolienabschnitt 15 wird hierdurch in die Formmulden 7 gesaugt und bleibt dort während der nachfolgenden Prozessschritte an Ort und Stelle gehalten.

Fig. 5 zeigt in Einzeldarstellung die Einführstation 3 nach Fig. 1 beim Einführen einer mit der Anzahl der Formmulden 7 korrespondierenden Anzahl von Katheter-Teilen 20. Zusätzlich zu dem eingelegten Basisfolienabschnitt 15 werden nun die Katheter-Teile 20 in die Formmulden 7 eingeführt. Dafür kommen verschiedene Möglichkeiten in Betracht. Vorliegend sind jeweils eine Katheter-Röhre 25 mit einem Anschlussstück 23 verbunden und werden gemeinsam von oben in die jeweilige Formmulde 7 eingeführt.

Nach Fig. 5 geschieht dies gleichzeitig. Es kann aber auch ein sequentielles bzw. zeitversetztes Einführen vorgesehen sein. Die zugehörigen Kopfstücke 24 werden von unten in die jeweilige Formmulde 7 eingeführt. Darüber hinaus können noch an dieser oder an einer oder mehreren weiteren Einführstationen weitere Einlegteile in die Formmulden eingelegt werden. Denkbar ist auch, dass beispielsweise nur jeweils ein aus Anschlussstück 23 und Kopfstück 24 gebildetes Teile-Paar oder eine Teile-Gruppe das betreffende einzulegende Katheter-Teil 20 im Sinne der erfindungsgemäßen Definition bildet, und dass weitere Teile wie die Katheter-Röhre 20 erst später nach dem nachfolgend beschriebenen Siegelprozess eingeführt werden.

Fig. 6 zeigt in Detaildarstellung die zweite Formstation 2 und die Siegelstation 4 nach Fig. 1 beim Formen und Aufsiegeln des Deckfolienabschnitts 16. In der zweiten Formstation 2 liegt die in Fig. 3 bereits gezeigt zweite Formplatte 9 mit ihren zweiten Formmulden 10 nach unten gewandt, wobei sich unterhalb davon zweite Einlegemittel 11 zum dehnungsfreien Einlegen des Deckfolienabschnitts 16 in die zweiten Formmulde 10 befinden. Die zweiten Einlegemittel 11 sind analog zu den ersten Einlegemitteln 8 (Fig. 4) als mit den zweiten Formmulden 10 korrespondierende Druckstempel 27 ausgeführt. Das dehnungsfreie Einlegen des Deckfolienabschnittes 16 in die zweiten Formmulden 10 mit dehnungsfreiem Nachführen von Folienmaterial aus der Folienbahn 14 erfolgt in gleicher Weise wie oben im Zusammenhang mit dem Basisfolienabschnitt 15 (Fig. 4) beschrieben.

Ein Unterschied besteht allerdings darin, dass die Deckfolienabschnitte 16 gemäß Ausführungsbeispiel nicht zusammenhängen bleiben, sondern dass in der zweiten Formstation beidseitig der zweiten Formplatte 7 jeweils Schneidmittel 18 angeordnet sind, welche den der Formplatte 7 zugeordneten Deckfolienabschnitt 16 aus der kontinuierlichen Folienbahn 14 ausschneiden und damit vereinzeln. Das Vereinzeln kann nach dem Formen vorgenommen werden. Alternativ kann es auch zweckmäßig sein, zunächst die Vereinzelung des Deckfolienabschnitts 16 mittels der Schneidmittel 18 durchzuführen, woraufhin dann erst die Formgebung erfolgt. Es ist dann ein ausreichendes Übermaß vorzusehen, aus dem heraus dann das beim Formgeben zusätzlich erforderliche Folienmaterial in die zweiten Formmulden 10 dehnungsfrei eingezogen werden kann.

Im Rahmen der Erfindung kann es zweckmäßig sein, auch die Basisfolienabschnitte 15 zu vereinzeln. Ebenso kann es in Betracht kommen, die Deckfolienabschnitte 16 ebenso wie die Basisfolienabschnitte 15 zumindest bis zur Trennstation als zusammenhängende, kontinuierliche Folienbahn 14' zu belassen.

In Fig. 6 ist noch erkennbar, dass die zweite Formplatte 9 mit zahlreichen Bohrungen versehen ist. Hierdurch kann Unterdruck in die zweiten Formmulden 10 eingeleitet werden, um den geformten Deckfolienabschnitt 16 nach dem Formvorgang in den Formmulden 10 zu halten. Hierdurch sind Haltemittel in Form einer Unterdruckzufuhr 13 gebildet. Analoges gilt für Haltemittel an den ersten Formplatten 6 in Form der weiter oben schon erwähnten Unterdruckfuhr 12.

Ausgehend von dem vorstehend beschriebenen Zustand wird nun die zweite Formplatte 9 mit dem darin gehaltenen und geformten Deckfolienabschnitt 16 entsprechend einem Pfeil 29 in eine Lage parallel zu den ersten Formplatten 6 aufgerichtet und dann entsprechend einem Pfeil 30 zur Siegelstation 4 verfahren. Dort trifft sie auf eine korrespondierende erste Formplatte 6, welche in vorstehend beschriebener Weise in ihren ersten Formmulden 7 einen Basisfolienabschnitt 15 sowie die genannten Katheter-Teile 20 aufgenommen hat und dort bereithält. Die zweite Formplatte 9 wird so auf die erste Formplatte 6 aufgelegt, dass der Deckfolienabschnitt 16 und der Basisfolienabschnitt 15 im Bereich der Siegelabschnitte 17 (Fig. 2) aufeinander aufliegen und dabei die eingelegten Katheter-Teile 20 umschließen. In diesem Zustand erfolgt nun der Siegelvorgang insbesondere unter Einwirkung der Siegelheizung 31 (Fig. 3), wobei dann die Siegelungen, genauer die Heißsiegelungen unter Verwendung eines Schmelzklebers an den Siegelabschnitten 17 und an den Siegelflächen 22 (Fig. 2) ausgeführt werden.

Nach dem Siegelvorgang wird die zweite Formplatte 7 wieder zurück in ihre Ausgangsposition der zweiten Formstation 2 verfahren.

Die nunmehr zusammengesiegelte Einheit aus Basisfolienabschnitt 15, Deckfolienabschnitt 16 und Katheter-Teilen 20 verbleibt in der ersten Formplatte 6 und wird gemeinsam mit dieser im Anschluss an den Siegelvorgang weiter zur nachfolgenden Trennstation 5 verfahren. Fig. 7 zeigt noch in perspektivischer Ansicht einen Teil dieser Trennstation 5. Hier ist eine Reihe von Folienmessern 31 vorgesehen, welche zwischen den Katheter-Teilen hindurch und auch außenseitig davon die doppelte Folienlage aus Basisfolienabschnitt 15 und Deckfolienabschnitt 16 durchtrennen und dadurch die eingesiegelten Katheter-Teile in die in Fig. 2 gezeigte Form vereinzeln.

## Patentansprüche

1. Vorrichtung zum Einsiegeln von zumindest einem Katheter-Teil (20) in eine Folienmanschette (21),
- umfassend eine erste Formstation (1), eine zweite Formstation (2), eine Einführstation (3) zum Einführen des Katheter-Teils (20) und eine Siegelstation (4) zum Aufsiegeln eines Basisfolienabschnitts (15) und eines Deckfolienabschnitts (16) aufeinander und auf Siegelflächen (22) des Katheter-Teils (20) unter Bildung der Folienmanschette (21),
- umfassend eine erste Formplatte (6) mit mindestens einer ersten Formmulde (7) für den Basisfolienabschnitt (15) und eine zweite Formplatte (9) mit mindestens einer zweiten Formmulde (10) für den Deckfolienabschnitt (16), wobei die erste Formmulde (7) und die zweite Formmulde (10) im Bereich des Basisfolienabschnitts (15) bzw. des Deckfolienabschnitts (16) eine eindimensional gekrümmte Querschnittsform aufweisen,
- und umfassend erste Einlegemittel (8) zum dehnungsfreien Einlegen des Basisfolienabschnitts (15) in die mindestens eine erste Formmulde (7), sowie zweite Einlegemittel (11) zum dehnungsfreien Einlegen des Deckfolienabschnitts (16) in die mindestens eine zweite Formmulde (10).

2. Vorrichtung nach Anspruch 1,
**dadurch gekennzeichnet, dass** in der ersten Formplatte (6) mehrere erste Formmulden (7) und in der zweiten Formplatte (9) eine korrespondierende Anzahl von zweiten Formmulden (10) ausgebildet sind.

3. Vorrichtung nach Anspruch 1 oder 2,
**dadurch gekennzeichnet, dass** die ersten Einlegemittel (8) und/oder die zweiten Einlegemittel (11) als mit den ersten bzw. zweiten Formmulden (7, 10) korrespondierende Druckstempel (27) ausgeführt sind.

4. Vorrichtung nach einem der Ansprüche 1 bis 3,
**dadurch gekennzeichnet, dass** die ersten und/oder zweiten Formplatten (6, 9) mit Haltemitteln zum Halten des eingelegten Basisfolienabschnitts (15) in der ersten Formmulde (7) bzw. des eingelegten Deckfolienabschnitts (16) in der zweiten Formmulde (10) versehen sind.

5. Vorrichtung nach Anspruch 4,
**dadurch gekennzeichnet, dass** die Haltemittel in Form einer Unterdruckzufuhr (12, 13) ausgeführt sind.

6. Vorrichtung nach einem der Ansprüche 1 bis 5,
**dadurch gekennzeichnet, dass** die ersten und/oder zweiten Formmulden (7, 10) in Form eines Halbzylinders ausgeführt sind.

7. Vorrichtung nach einem der Ansprüche 1 bis 6,
**dadurch gekennzeichnet, dass** Schneidmittel (18) zur Vereinzelung des Basisfolienabschnitts (15) bzw. des Deckfolienabschnitts (16) aus einer kontinuierlichen Folienbahn (14) vorgesehen und insbesondere in zumindest einer der beiden Formstationen (1, 2) angeordnet sind.

8. Vorrichtung nach einem der Ansprüche 1 bis 7,
**dadurch gekennzeichnet, dass** in zumindest einer der beiden Formplatten (6, 9) eine Siegelheizung (31) integriert ist.

9. Vorrichtung nach einem der Ansprüche 1 bis 8,
**dadurch gekennzeichnet, dass** die Vorrichtung eine Trennstation (5) zum Vereinzeln von mehreren eingesiegelten Katheter-Teilen (20) umfasst.

10. Verfahren zum Einsiegeln eines Katheter-Teils (20) in eine Folienmanschette (21) mittels einer Vorrichtung nach einem der Ansprüche 1 bis 9, umfassend folgende Verfahrensschritte:
- In der ersten Formstation (1) wird ein Basisfolienabschnitt (15) in mindestens eine erste Formmulde (7) einer ersten Formplatte (6) dehnungsfrei eingelegt,
- In einer Einführstation (3) wird mindestens ein Katheter-Teil (20) bei eingelegtem Basisfolienabschnitt (15) in eine korrespondierende erste Formmulde (7) eingeführt,
- In der zweiten Formstation (2) wird ein Deckfolienabschnitt (16) in mindestens eine zweite Formmulde (10) einer zweiten Formplatte (9) dehnungsfrei eingelegt,
- In der Siegelstation (4) wird das Katheter-Teil (20) in eine Folienmanschette (21) eingesiegelt, indem die erste Formplatte (6) und die zweite Formplatte (9) bei eingelegtem Basisfolienabschnitt (15), bei eingelegtem Deckfolienabschnitt (16) und bei eingeführtem Katheter-Teil (20) zusammengeführt werden, wobei der Basisfolienabschnitt (15) und der Deckfolienabschnitt (16) an gemeinsamen Siegelabschnitten (17) aufeinander gesiegelt und auf Siegelflächen (22) des Katheter-Teils (20) unter Bildung der Folienmanschette (21) aufgesiegelt werden.
